# FASCICULE DE BREVET EUROPEEN

(11) **EP 2 512 395 B1**
(45) Date de publication et mention de la délivrance du brevet: **26.03.2014**
(21) Numéro de dépôt: 10798050.0
(22) Date de dépôt: 15.12.2010
(51) Int. Cl.: A61H 5/00, A61B 3/113

(54) **DISPOSITIF D'ENTRAINEMENT ET/OU DE RÉÉDUCATION DE LA MOTRICITÉ BINOCULAIRE D'UN PATIENT**
VORRICHTUNG ZUR AUSLÖSUNG UND/ODER REHABILITATION DER ZWEIÄUGIGEN BEWEGUNGSFÄHIGKEIT EINES PATIENTEN
DEVICE FOR CAUSING AND/OR REHABILITATING BINOCULAR MOTIVITY OF A PATIENT

(30) Priorité: 15.12.2009 FR 0958994
(43) Date de publication de la demande: 24.10.2012
(73) Titulaire: Centre National de la Recherche Scientifique (C.N.R.S.), 75016 Paris (FR)
(72) Inventeur: KAPOULA, Zoi, F-75015 Paris (FR); YANG, Qing, F-94140 Alfortville (FR); EGGERT, Thomas, 81377 Munich (DE); DAUNYS, Gintautas, 77171 Siauliai (LT); ORSSAUD, Christophe, F-75015 Paris (FR)
(74) Mandataire: Regimbeau
(86) Numéro de dépôt international: PCT/EP2010/069830
(87) Numéro de publication internationale: WO 2011/073288

(56) Documents cités:
- DE-A1- 2 828 532
- THURID SANDER: "Unterschiedliche Kodierung und Interaktion langsamer konjugierter und diskonjugierter Augenbewegungen im Raum", INTERNET CITATION, 1 janvier 2006 (2006-01-01), page 117PP, XP007914029, Extrait de l'Internet: URL:http://deposit.d-nb.de/cgi-bin/dokserv ?idn=984117326&dok_var=d1&dok_e xt=pdf&filename=984117326.pdf [extrait le 2010-07-20]

## Description

### DOMAINE TECHNIQUE GENERAL

L'invention concerne un dispositif de rééducation et/ou d'entrainement de la motricité binoculaire. Et l'invention trouve avantageusement application dans le traitement des pathologies ayant un lien avec la motricité binoculaire.

### ETAT DE LA TECHNIQUE

L'entrainement et/ou la rééducation de la motricité binoculaire consiste à faire effectuer des exercices à un patient au moyen de divers dispositifs tels que des barres de prismes, synoptophore, etc.

Ces techniques sont toutefois limitées.

En effet, elles sont subjectives puisqu'elles sont conditionnées par la réponse du patient à l'exercice en question pour passer à l'exercice suivant : est-ce que le patient voit double par exemple. Ceci implique qu'il n'y a pas de mesures objectives car les progrès réalisés par le patient ne sont mesurables qu'au travers des échanges entre le praticien et le patient.

En outre, ces techniques sont statiques et ne peuvent pas rééduquer/entrainer la trajectoire des mouvements des yeux notamment lorsque certaines pathologies sont liées au mouvement des yeux.

De ce fait, les résultats de l'entrainement/ rééducation orthoptique peuvent être aléatoires, dépendre du praticien et de la coopération du sujet.

Le document THURID SANDER: "Unterschiedliche Kodierung und Interaktion langsamer konjugierter und diskonjugierter Augenbewegungen im Raum", Inauguraldissertation zur Erlangung der Doktorwürde der Universität zu Lübeck, Lübeck 2006, décrit un dispositif de rééducation et/ou d'entraînement de la motricité binoculaire par stimulation sensorielle d'un patient, comprenant : un support plan défini par un axe de symétrie longitudinal, une pluralité de moyens de diffusion d'au moins un stimulus sensoriel, lesdits moyens de diffusion comprenant des moyens de visualisation d'un stimulus visuel, et un système de commande au moins destiné à commander la diffusion dudit stimulus visuel.

### PRESENTATION DE L'INVENTION

L'invention permet de remédier aux inconvénients précités.

A cet effet, l'invention concerne un dispositif de rééducation et/ou d'entrainement de la motricité binoculaire par stimulation sensorielle d'un patient comprenant : un support plan défini par un axe de symétrie longitudinal, le support comprenant une pluralité de moyens de diffusion d'au moins un stimulus sensoriel, lesdits moyens de diffusion comprenant des moyens de visualisation d'un stimulus visuel et des moyens de diffusion d'un stimulus auditif adjacents aux moyens de visualisation d'un stimulus visuel, les moyens de diffusion étant arrangés de manière régulière selon des arcs d'iso-vergence disposés le long du support plan ; un système de commande au moins destiné à commander la diffusion dudit au moins stimulus sensoriel.

Le dispositif de l'invention permet la rééducation et/ou l'entrainement de la motricité binoculaire sur l'axe horizontal, vertical et en profondeur et répond à une demande clinique forte (orthoptie, optométrie hospitalière ou libérale, optique ergonomie visuelle).

Le dispositif de l'invention permet en outre de stimuler la synergie physiologique, c'est-à-dire l'ensemble des indices visuels en même temps : disparité binoculaire, convergence et accommodation du cristallin. Le dispositif de l'invention permet de stimuler la vision telle qu'elle est au quotidien en combinant l'ensemble des ressources physiologiques.

De manière avantageuse, sur chaque arc d'isovergence, les moyens de génération d'un stimulus visuel et les moyens de diffusion d'un stimulus auditif sont disposés de sorte qu'à chaque moyen de visualisation d'un stimulus visuel est associé un moyen de diffusion d'un stimulus auditif.

Le couplage des moyens de stimulation visuelle et auditive permet une stimulation simultanée de plusieurs modalités sensorielles, particulièrement important dans les cas de déficience visuelle comme la dégénérescence maculaire liée à l'âge basse vision (DMLA basse vision).

D'autres aspects du dispositif selon l'invention sont les suivants :
- le dispositif comprend plus de deux arcs d'iso-vergence, préférentiellement quatre arcs d'iso-vergence ;
- chaque arc d'iso-vergence comprend au moins trois moyens de génération d'un stimulus sensoriel, préférentiellement neuf moyens de génération d'au moins un stimulus sensoriel ;
- le support est trapézoïdal ou triangulaire ;
- le support peut être un panneau rabattable, un éventail pliable ou en plusieurs parties emboitables ;
- le dispositif comprend un premier arc d'iso-vergence à une distance de 20 cm du patient, un second arc d'iso-vergence à une distance de 40 cm du patient, un troisième arc d'iso-vergence à une distance de 70 cm du patient, un quatrième arc d'iso-vergence à une distance de 150 cm du patient ;
- les moyens de visualisation comprennent des diodes électroluminescentes ;
- les moyens de diffusion d'un stimulus auditif comprennent des hauts-parleurs ;
- les moyens de diffusion d'un stimulus auditif étant adaptés pour diffuser dans certains cas un stimulus auditif de fréquences distinctes d'un arc d'iso-vergence à l'autre ;
- le dispositif comprend des moyens de couplage à un dispositif d'acquisition des mouvements des yeux du patient.

Selon un second aspect, il est divulgué un procédé de rééducation et/ou d'entrainement de la motricité binoculaire au moyen d'un dispositif de rééducation selon le premier aspect de l'invention.

Le procédé de rééducation/entrainement permet la rééducation/l'entrainement de la motricité binoculaire sur l'axe horizontal, vertical et en profondeur.

La rééducation/entrainement effectué par le procédé améliore la qualité de la vision de l'espace tridimensionnel mais aussi le déploiement des capacités attentionnelles et cognitives.

Le procédé est particulièrement adapté aux troubles suivants : déficit de la vergence oculomotrice, vertiges, céphalées, strabisme, troubles neuro-ophtalmologiques, maladies neuro-dégénératives et vieillissement, hyperactivité, dyslexie, ainsi qu'à la rééducation après chirurgie réfractive et la rééducation en basse vision, acouphènes liés aux problèmes de vergence et de l'interaction saccade et vergence ; pourrait aussi servir d'outil de stimulation neurosensorielle alternante (gauche.droite) et à des profondeurs différentes pour des psychothérapeutes dans le domaine de psychopathologie et psychiatrie.

De manière avantageuse en couplant le dispositif de l'invention avec un dispositif d'enregistrement du mouvement des yeux il est possible de mesurer de manière objective les progrès réalisés par le patient rééduqué/entrainé, en particulier, la modification physiologique de la trajectoire du mouvement des yeux, leur ampleur et précision par rapport aux stimuli sensoriels.

Le dispositif de l'invention inclura une brochure - utilisateur de présentation des différents protocoles d'entraînement, ainsi qu'un service d'échange sur des pathologies particulières avec l'inventeur principal de la présente demande.

### PRESENTATION DES FIGURES

D'autres caractéristiques et avantages de l'invention ressortiront encore de la description qui suit laquelle est purement illustrative et non limitative et doit être lue en regard des dessins annexés sur lesquels :
- la figure 1 illustre un dispositif de rééducation et/ou d'entrainement de la motricité binoculaire d'un patient selon l'invention;
- la figure 2 illustre, selon un premier mode de réalisation non couvert par les revendications, une vue de dessus d'un support du dispositif de rééducation et/ou d'entrainement de la motricité binoculaire d'un patient (moyens de visualisation des stimuli visuels) ;
- la figure 3 illustre, selon un second mode de réalisation, une vue de dessus d'un support du dispositif de rééducation et/ou d'entrainement de la motricité binoculaire d'un patient selon l'invention (moyens de visualisation des stimuli visuels et moyens de génération des stimuli auditifs) ;
- les figure 4a, 4b, 4c illustrent des vues de dessus du support selon l'invention dans plusieurs configurations d'utilisation ;
- les figures 5a, 5b et 5c illustrent le support de l'invention selon plusieurs états successifs d'une configuration d'activation d'un stimulus sensoriel selon une séquence dite « *gap* ») ;
- la figure 6 illustre les durées d'activation successives d'un stimulus sensoriel selon les états des figures 5a, 5b et 5c ;
- les figures 7a, 7b et 7c illustrent le support de l'invention selon plusieurs états successifs d'une configuration d'activation d'un stimulus sensoriel selon une séquence dite *« overlap* » de type simple saut ;
- la figure 8 illustre les durées d'activation successives d'un stimulus sensoriel selon les états des figures 7a, 7b et 7c ;
- les figures 9a, 9b, 9c et 9d illustrent le support de l'invention selon plusieurs états successifs d'une configuration d'activation d'un stimulus sensoriel selon une séquence dite « *gap* » de type double saut ;
- la figure 10 illustre les durées d'activation successives d'un stimulus sensoriel selon les états des figures 9a, 9b, 9c et 9d ;
- les figures 11a, 11b, 11c illustrent le support de l'invention selon plusieurs états successifs d'une configuration d'activation d'un stimulus sensoriel selon une séquence dite *« overlap* » de type double saut ;
- la figure 12 illustre les durées d'activation successives d'un stimulus sensoriel selon les états des figures 11a, 11b, 11c ;
- les figures 13a, 13b et 13c illustrent l'évolution de la réponse d'un patient à des séquences d'entrainement diffusées au moyen du dispositif de rééducation et/ou d'entrainement de la motricité binoculaire d'un patient selon l'invention.

### DESCRIPTION DETAILLEE DE L'INVENTION

### Description du dispositif

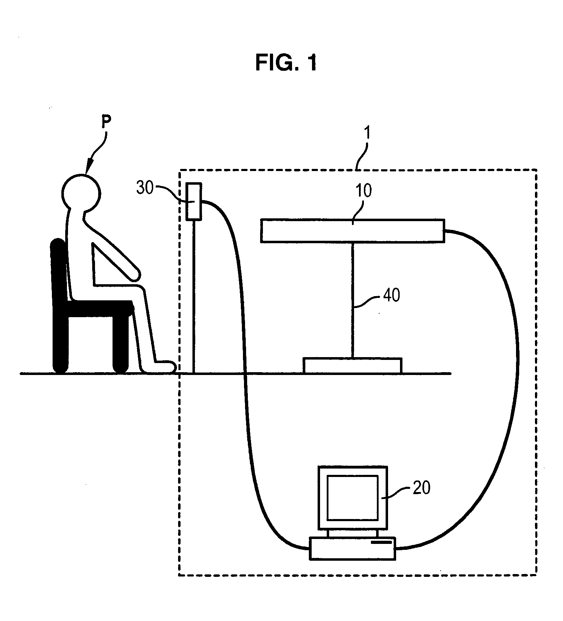

La figure 1 illustre un dispositif de rééducation et/ou d'entrainement de la motricité binoculaire d'un patient conforme à la présente invention.

Le dispositif 1 comprend un support 10 sur lequel sont arrangés des moyens de diffusion d'au moins un stimulus sensoriel, un système de commande 20 destiné à commander au moins la diffusion d'au moins un stimulus sensoriel. Le système de commande 20 est par exemple un ordinateur de type PC connecté au support (en particulier aux moyens de diffusion d'un stimulus sensoriel) via une interface électronique-informatique (non représentée).

On précise que l'on entend, dans le cadre de la présente invention, par stimulus sensoriel, soit un stimulus visuel, soit un stimulus auditif soit la combinaison d'un stimulus visuel et d'un stimulus auditif.

On précise également que l'on entend par diffusion d'au moins un stimulus sensoriel la visualisation d'un stimulus visuel, la diffusion d'un stimulus auditif soit ensemble la visualisation d'un stimulus visuel et la diffusion d'un stimulus auditif.

Le support 10 peut être disposé sur une table (non représentée) ou être monté sur un pied 40. Le pied 40 du support peut être réglable en hauteur et/ou en inclinaison.

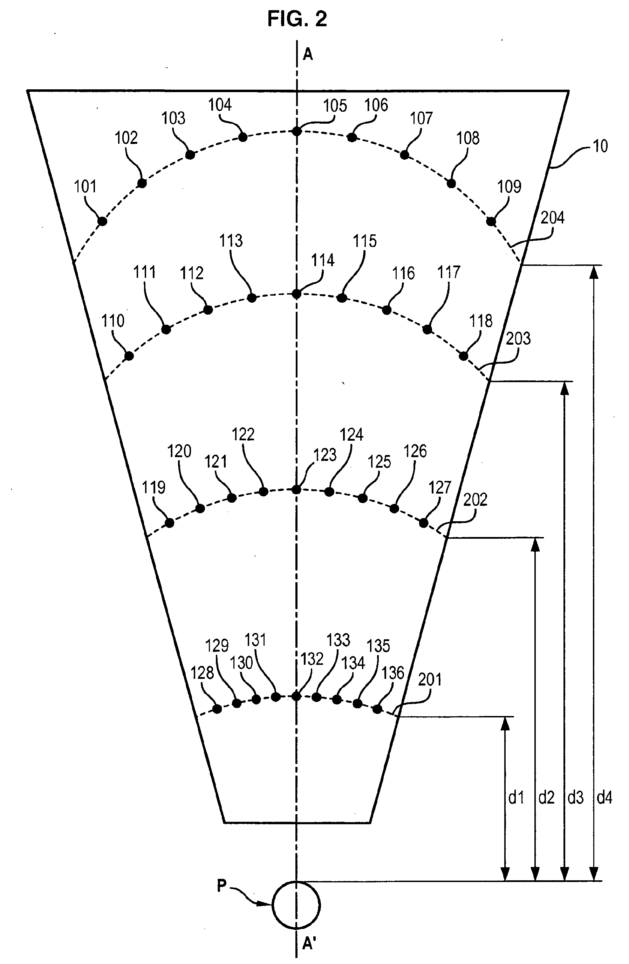

La figure 2 illustre une vue de dessus du support 10 selon un premier mode de réalisation non couvert par les revendications.

Le support 10 est un support plan défini par un axe de symétrie AA' longitudinal et comprend des moyens de visualisation 101 à 136 d'un stimulus visuel arrangés de manière régulière selon des arcs d'iso-vergence 201 à 204 disposés le long du support. On a représenté de manière non limitative sur la figure 2 le support 10 en position horizontal.

On précise que l'on entend par arc d'iso-vergence des arcs tels que l'angle de vergence des yeux requis pour fixer chaque stimulus sensoriel sur un arc donné est le même. Cet angle est défini par rapport à la position du patient P à l'extrémité du support 10.

Le support 10 comprend de préférence plus de deux arcs d'isovergence et très préférentiellement quatre arcs d'iso-vergence 201, 202, 203, 204. Dans ce cas là, le premier arc d'iso-vergence 201 est destiné à être une distance d1 de 20 cm du patient P, le second arc d'iso-vergence 202 est destiné à être à une distance d2 de 40 cm du patient P, le troisième arc d'iso-vergence 203 est destiné à être à une distance d3 de 70 cm du patient P, le quatrième arc d'iso-vergence 204 est destiné à être à une distance d4 de 150 cm du patient P.

Le support 10 peut être triangulaire (non représenté) ou trapézoïdal.

Dans le cas d'un support trapézoïdal, la petite base est de dimension comprise entre 20 cm et 30 cm, de préférence 24 cm et la grande base est de dimension comprise entre 110 cm et 130 cm, de préférence 120 cm. En outre, le patient P est destiné à être situé du côté de la petite base du support.

Dans le cas d'un support triangulaire, le patient P est destiné à être placé au niveau d'un sommet du triangle et deux côtés s'étendent du sommet selon un angle d'ouverture compris entre 50° et 60°, typiquement 54°. Les deux côtés s'étendant à partit du sommet où se situe le patient P sont de longueurs identiques comprise entre 160 cm et 190 cm, typiquement 170 cm.

Afin d'avoir un support 10 léger et pliable, on peut prévoir qu'il soit sous la forme d'un éventail chinois. Un tel support est divisé en plusieurs sous-bandes séparées par des lignes de pliage. Ce support comprend également des baleines parallèles aux lignes de pliage.

Dans le cas où le support est sous la forme d'un éventail chinois, les moyens de visualisation des stimuli visuels seront arrangés sur les baleines de l'éventail. Ainsi, comme on le comprend, sous cette forme ci, le support 10 est d'un encombrement faible et est facilement transportable.

De manière avantageuse, le support peut être formé d'un assemblage de plusieurs éléments, chaque élément correspondant à un arc d'isovergence. Les éléments sont alors emboitables. Ceci contribue également à avoir un support d'encombrement faible lorsqu'il n'est pas utilisé et est facilement transportable.

Les moyens de visualisation 101-136 d'un stimulus visuel sont de préférence des diodes électroluminescentes (en anglais, « *Light Emitting Device* », (LED)). Dans ce cas, les LED doivent être inoffensives pour les yeux (par exemple LED rouges, longueur d'onde 620-625 nm ; 20mA sous 2V ; 100-200 mcd).

Bien entendu on peut prévoir que les moyens de visualisation d'un stimulus visuel sont formés par une source de lumière émettant dans le domaine du visible.

Comme on l'a déjà mentionné, les moyens de visualisation sont disposés régulièrement sur les arcs d'iso-vergence. Chaque arc d'isovergence comprend plusieurs moyens de visualisation d'un stimulus visuel, typiquement au moins trois moyens de visualisation d'un stimulus visuel, préférentiellement plus de trois moyens de visualisation d'un stimulus visuel et avantageusement neuf moyens de visualisation d'un stimulus visuel 101 à 109, 110 à 118, 119 à 117, 128 à 136.

Le pas entre chaque moyen de visualisation est compris entre 2° et 8° d'angle visuel, typiquement 5°.

Les arcs d'iso-vergence sont disposés symétriquement par rapport à l'axe de symétrie AA' longitudinal du support 10 et sont tels que la concavité est dirigée vers le patient (ou vers le sommet du triangle où est situé le patient lorsque le support est triangulaire ou encore vers la petite base où est situé le patient lorsque le support est trapézoïdal).

En plus des moyens de visualisation d'un stimulus visuel, selon l'invention on prévoit également des moyens de diffusion d'un stimulus auditif.

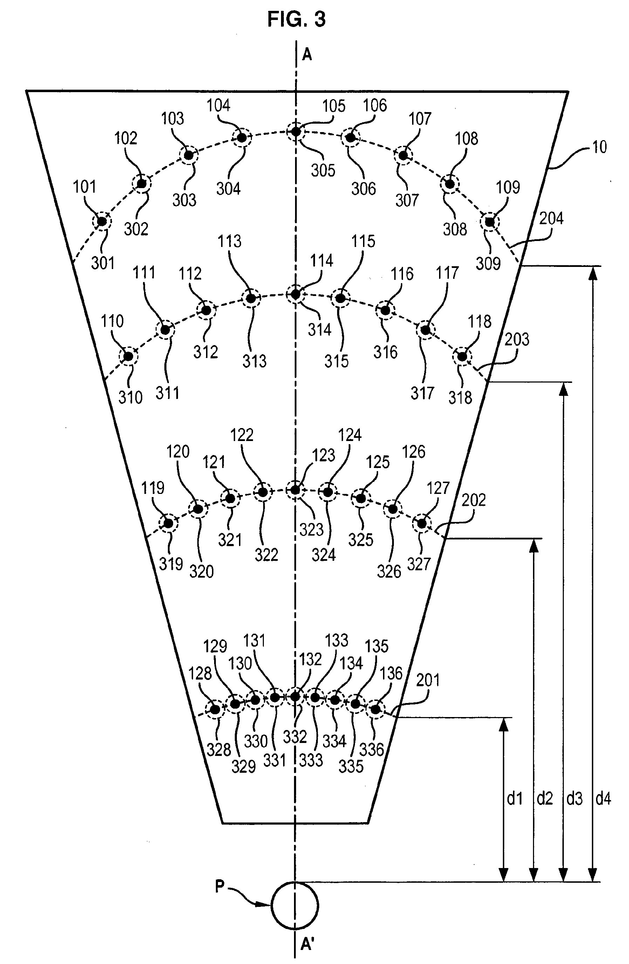

La figure 3 illustre une vue de dessus du support selon un second mode de réalisation conforme à la présente invention.

Le support de la figure 3 comprend - en plus du support présenté ci-dessus - des moyens de diffusion 301 à 336 d'un stimulus auditif.

De tels moyens de diffusion 301 à 336 d'un stimulus auditif sont par exemple des haut-parleurs et sont disposés près d'un stimulus visuel. Par conséquent, le nombre de stimuli auditifs est égal au nombre de stimuli visuels (par exemple neuf pour chaque arc d'iso-vergence).

La diffusion d'un stimulus auditif est effectuée par le système de commande 20.

En outre, le dispositif de rééducation/entrainement peut comprendre des moyens d'acquisition 30 des mouvements des yeux du patient P, à savoir, saccades oculaires ou mouvements de vergence seuls ou combinés.. De tels moyens d'acquisition sont par exemple constitués par un électro-oculographe ou un vidéo-oculographe.

De tels moyens 30 servent à enregistrer le mouvement des yeux du patient P et contribuent à optimiser la rééducation (comme il sera discuté ci-dessous).

Un appareil d'enregistrement du mouvement des yeux peut être du type connu « EOG blue gain, Cambridge Instrument ». Il peut s'agit aussi de tout autre système de vidéo-oculographie de type connu (par exemple Tobii™, Chronos™, Eyelink™, SMI™, See Eye Cam de : *University of Munich Hospital*)*.*

Le dispositif ci-dessus décrit peut être prévu sous une forme miniaturisé (avec seulement trois arcs d'iso-vergences) permettant un usage à domicile.

### Application à la rééducation/entrainement de la motricité binoculaire d'un patient

Le support 10 présenté ci-dessus dans le premier et le second mode de réalisation commandé par le système de commande 20 permet la génération de plusieurs paradigmes d'entrainement visuo-moteur (stimulation visuelle et éventuellement auditive) à l'attention du patient P situé à l'extrémité du support 10, c'est-à-dire vers le sommet du triangle où est situé le patient lorsque le support est triangulaire ou encore vers la petite base où est situé le patient lorsque le support est trapézoïdal.

Les séquences d'entrainement sont générées par une application implémentée dans le système de commande 20. Une telle application comporte plusieurs menus pour différents types d'entrainement selon la pathologie ou le dysfonctionnement du patient à rééduquer/entrainer.

Les séquences permettent un entrainement des mouvements combinés en direction (selon une direction transversale par rapport à l'axe de symétrie AA' longitudinal du support) et en profondeur (selon une direction définie par l'axe de symétrie AA' longitudinal du support), ou des mouvements en profondeur seulement ou encore des mouvements en direction seulement.

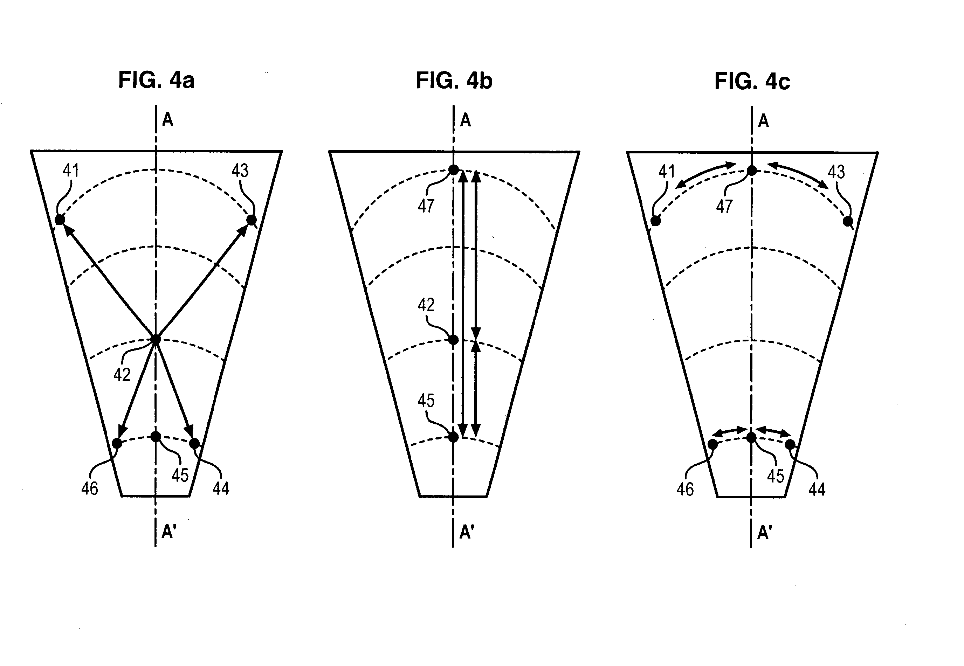

Les figures 4a, 4b, 4c illustrent le résultat de la commande d'un ou plusieurs moyens de visualisation de stimuli visuels et éventuellement de diffusion des stimuli auditifs correspondants.

Sur ces figures, un point correspond à l'activation d'un stimulus visuel ou bien d'un stimulus auditif ou bien d'un stimulus visuel plus le stimulus auditif correspondant.

Les figures 4a, 4b et 4c illustrent l'état du support lorsqu'une séquence de rééducation/d'entrainement par des mouvements combinés saccades et vergences, par des vergences pures ou par des saccades pures respectivement, est générée.

La séquence débute par l'activation d'un stimulus « initial » (visuel seul ou visuel plus auditif) au centre d'un arc d'iso-vergence. Puis, après quelques secondes, typiquement 2,5 secondes, un stimulus « cible » est activé.

De manière alternative ou complémentaire, pour chaque séquence lorsque l'on est dans un cas où l'on souhaite l'activation d'un stimulus cible visuel plus un stimulus auditif on peut prévoir que les activations sont simultanées ou différées. Dans le cas d'une activation différée, un intervalle compris entre 50 et 150 ms est appliqué (auditif puis visuel).

Le stimulus « cible » peut être décalé transversalement par rapport à l'axe de symétrie AA' longitudinal du support et être sur un autre arc d'isovergence que le stimulus « initial » (c'est-à-dire, par exemple, activation du stimulus latéral 41 ou 43 après activation du stimulus 42 ou encore activation du stimulus latéral 44 ou 46 après activation du stimulus 42). Une telle stimulation provoque chez le patient un mouvement combiné de saccade et de vergence (voir la figure 4a)

Le stimulus « cible » peut être au centre d'un autre arc d'iso-vergence (c'est-à-dire activation du stimulus central 45 après activation du stimulus central 42 ou activation du stimulus 47 après activation du stimulus 42). Une telle stimulation provoque chez le patient un mouvement de vergence (voir la figure 4b).

Le stimulus « cible » peut être décalé transversalement par rapport à l'axe de symétrie AA' longitudinal du support sur le même arc d'iso-vergence que le stimulus « initial » (c'est-à-dire activation du stimulus latéral 41 ou 43 après activation du stimulus central 47 ou encore activation du stimulus latéral 44 ou 46 après activation du stimulus 45). Une telle stimulation provoque chez le patient une saccade (voir la figure 4c).

On décrit ci-dessous des séquences utilisées pour la rééducation d'un patient.

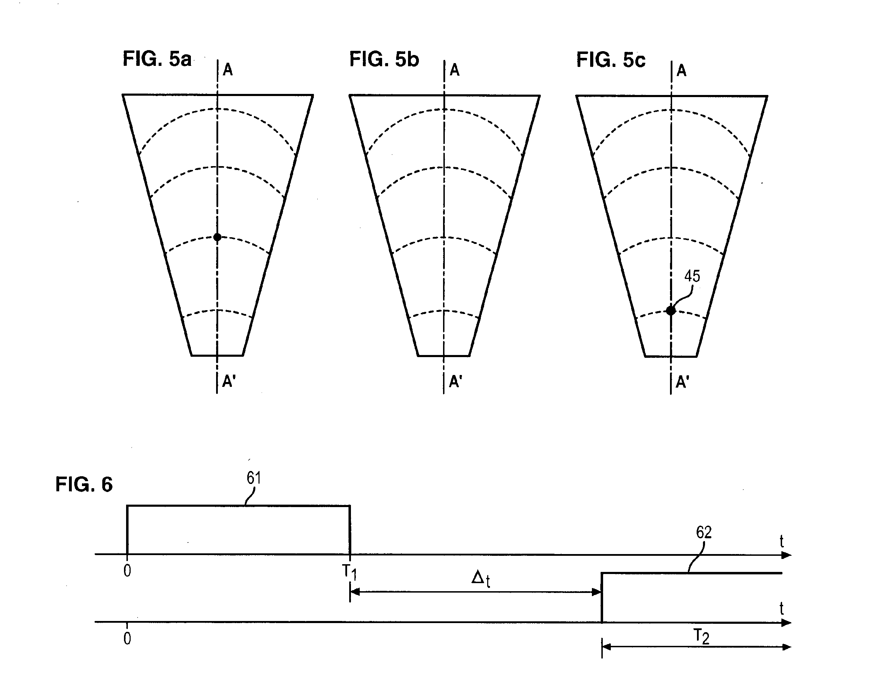

Les figures 5a, 5b, 5c et 6 illustrent une séquence dite *« gap* » (séquence avec un intervalle) de type simple saut.

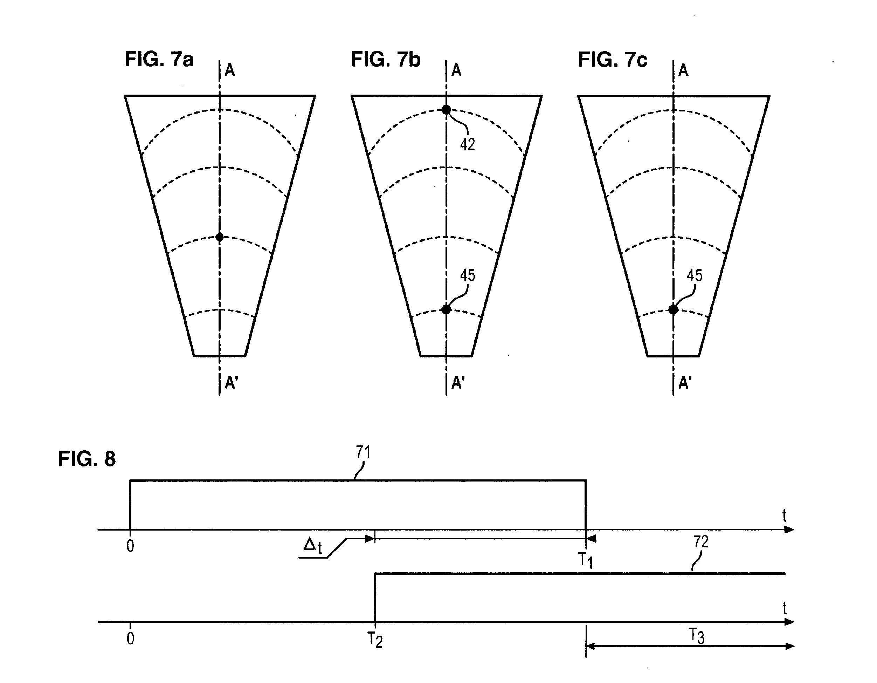

Les figures 7a, 7b, 7c et 8 illustrent une séquence dite *« overlap* » (séquence avec un chevauchement) de type simple saut.

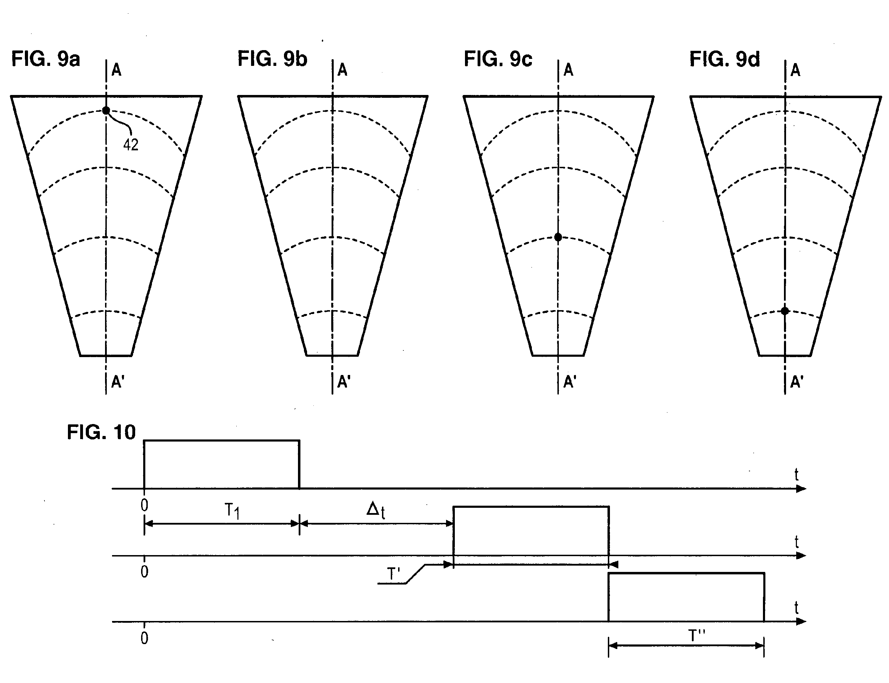

Les figures 9a, 9b, 9c et 10 illustrent une séquence dite « *gap* » de type double saut.

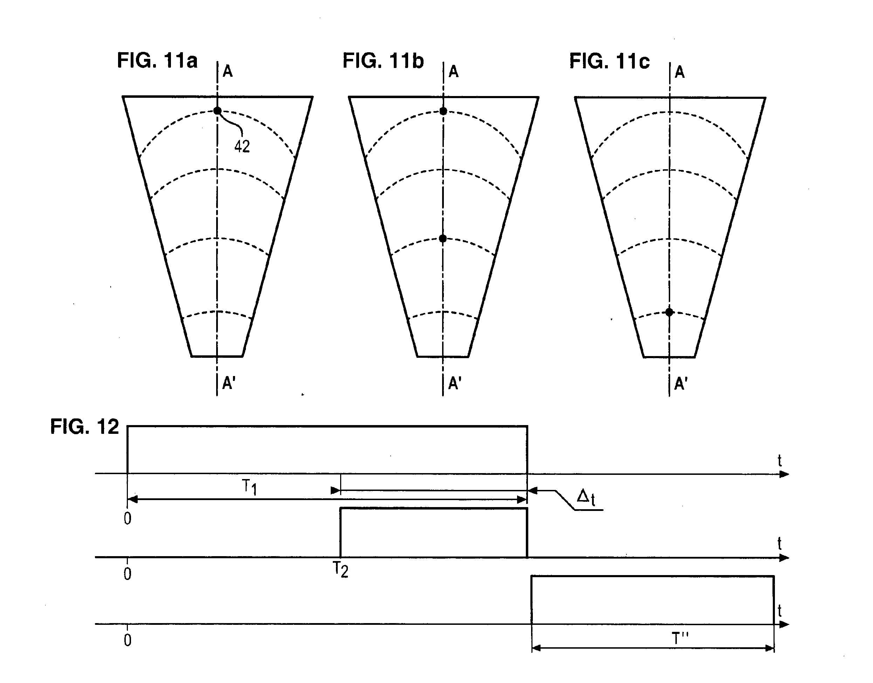

Les figures 11a, 11b, 11c et 12 illustrent une séquence dite *« overlap* » de type double saut.

La séquence dite « *gap »* illustrée sur les figures 5a, 5b, 5c et 6 permet de provoquer chez le patient un mouvement de pure vergence (selon une direction parallèle à l'axe de symétrie AA' longitudinal du support).

La figure 6 illustre les durées d'activation d'un stimulus sensoriel (soit visuel seul, soit auditif seul, soit auditif et visuel) au cours de la séquence « gap » simple saut.

Un stimulus « initial » situé au centre d'un arc d'iso-vergence est activé pendant une durée T1 comprise entre 1000 et 2000 ms, de préférence 1500 ms, ensuite pendant une durée Δt aucun stimulus est activé. A l'issue de la durée Δt comprise entre 100 et 400 ms, de préférence 200 ms, un stimulus « cible » central d'un arc d'iso-vergence différent de celui activé pendant la première durée T1 est activé pendant une durée T2 comprise entre 1000 et 2000 ms, de préférence 1500 ms.

La séquence « *overlap* » illustrée sur les figures 7a, 7b, 7c et 8 permet de provoquer chez le patient, de manière alternative à la séquence « *gap* », un mouvement de pure vergence mais avec recouvrement des stimulations.

Un stimulus « initial » situé au centre d'un arc d'iso-vergence est activé pendant une durée T1 comprise entre 1000 et 2000 ms, de préférence 1500 ms, ensuite pendant une durée Δt, le stimulus « initial » reste activé en même temps qu'un stimulus « cible » situé sur un autre arc d'iso-vergence est activé. A l'issu de la durée Δt comprise entre 100 et 400 ms, de préférence 200 ms, seul le stimulus « cible » est activé pendant une durée T3 comprise entre 1000 et 2000 ms, de préférence 1500 ms.

Les séquences « *gap »* et *« overlap* » peuvent être du type double saut.

La séquence illustrée les figures 9a, 9b, 9c et 9d est une séquence « *gap* » du type double saut. La figure 10 illustre les durées d'activation d'un stimulus sensoriel (soit visuel soit auditif et visuel) au cours de la séquence *« gap* » double saut.

A l'issu de la durée Δt de la séquence, le stimulus cible n'est activé que pendant une durée courte T' (entre 150-200 ms) ; puis est suivi de l'activation d'un autre stimulus cible pendant une durée T" de 800 à 1800 ms de préférence 1300 ms. L'emplacement du deuxième stimulus demande donc un agrandissement du mouvement initial provoqué par le premier stimulus.

La séquence illustrée sur les figures 11a, 11b et 11c est une séquence « *overlap* » du type double saut. La figure 12 illustre les durées d'activation d'un stimulus sensoriel (soit visuel soit auditif et visuel) au cours de la séquence « *overlap* » double saut.

Dans cette configuration de la séquence « *d'overlap* », le premier stimulus cible n'est activé que pendant une durée courte T2 (entre 150-200 ms) ; à l'issu de cette durée Δt un autre stimulus cible est activé pendant une durée T" de 800 à 1800 ms de préférence 1300 ms. Comme pour la séquence « *gap* » du type double saut, l'emplacement du deuxième stimulus cible demande un agrandissement du mouvement initial.

D'autres protocoles d'entraînement de type « *gap* » ou *« overlap* » double saut demanderont un raccourcissement du mouvement initial (non montrés).

Le protocole d'entrainement basé sur des séquences du type double saut est plus drastique et très efficace car il provoque la mise en place par le système nerveux central d'une régulation adaptative nouvelle (élaboration de la commande motrice par rapport au stimulus final et non pas par rapport au stimulus transitoire initial). La technique peut être particulièrement utile dans le cas de pathologies neuro-ophtalmologiques (parésies oculomotrices) et chez des patients avec basse vision, DMLA.

Au cours de la rééducation, grâce aux moyens 30 d'acquisition du mouvement des yeux, il est possible de suivre au cours de la rééducation le mouvement des yeux et d'adapter, le cas échéant, les séquences. En particulier, il est possible par une analyse en temps réel - au cours de la rééducation - de déterminer les paramètres (temporels et spatiaux) de stimulation visuelle et/ou acoustique optimaux pour l'entraînement de chaque patient. Par exemple, dans le protocole de double saut décrit ci-dessus, la valeur de la durée de présentation du premier stimulus est approximative et choisie par défaut sur la base de la recherche physiologique préalable. Elle pourrait être ajustée au patient lui-même en mesurant au préalable le temps de latence de sa vergence en faisant quelques séquences de type simple saut. Ainsi le protocole d'entraînement pourrait être personnalisé.

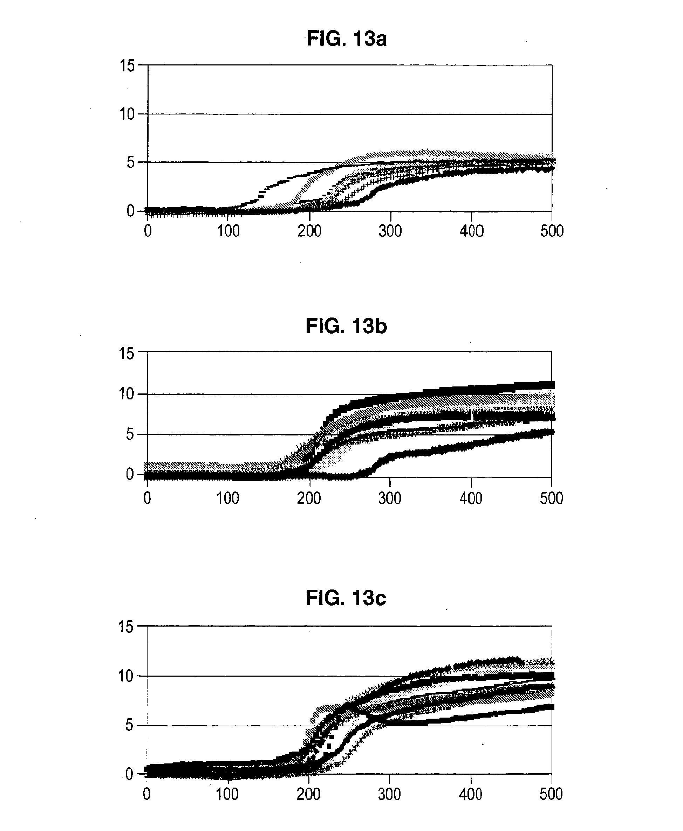

Les figures 13a, 13b et 13c illustrent des résultats obtenus sur un patient qui a été rééduqué selon le procédé décrit (en simple saut avec une séquence gap). Il s'agit d'un adolescent présentant des troubles de l'équilibre, des vertiges et des céphalées, particulièrement en fin de journée. L'examen, réalisé par le service ORL de l'Hôpital Robert Debré, a révélé une fonction vestibulaire normale, ce qui pose le problème de l'origine du vertige. L'examen clinique orthoptique a soulevé des suspicions pour une anormalité de la capacité de cet adolescent à générer des mouvements de vergence (divergence et convergence) correctement. Toutefois, cet examen ne permettait pas une quantification des anormalités des mouvements.

L'adolescent devait fixer une cible centrale à 68 cm, puis cette cible s'éteignait, et une cible apparaissait aléatoirement plus près (25 cm) ou plus loin (150 cm), ce qui provoquait des mouvements de convergence et de divergence respectivement. L'adolescent a réalisé trois blocs d'essais successifs (d'environ 4 minutes chacun), séparé par une pause d'environ une minute entre les blocs.

Les figures 13a, 13b et 13c montrent pour chacun des trois blocs les trajectoires de la convergence des yeux, pour les mouvements allant de 68 cm (correspondant à un angle de vergence de 5°) à 25 cm (correspondant à un angle de vergence de 13,68°). Ces enregistrements ont été effectuées avec un appareil de vidéoculographie « Chronos 2D/3D Eye Tracker™ ».

On observe pour le premier bloc (voir la figure 13a) une lenteur dans l'initiation des mouvements (temps moyen 200-250 ms) ainsi qu'une amplitude du mouvement faible (environ 5° alors que l'amplitude requise est de 8,68°). Lors du deuxième bloc (voir la figure 13b), l'initiation est accélérée (temps moyen 180-200 ms) et l'amplitude du mouvement augmente (côtoie les 8,68° requis). Enfin, l'on observe dans le troisième bloc (voir la figure 13c) un maintien des améliorations remarquées dans le deuxième bloc.

Ainsi, la répétition de ces mouvements avec des paramètres spatio-temporels similaires à ceux de notre dispositif met en évidence une amélioration objective de l'initiation et de la trajectoire des vergences. Ce protocole d'entraînement (séquence gap du type simple saut) était suffisant pour cet adolescent présentant une faiblesse de la vergence sans pathologie vestibulaire oculaire ou neurologique.

## Revendications

1. Dispositif de rééducation et/ou d'entrainement de la motricité binoculaire par stimulation sensorielle d'un patient comprenant :
- un support plan (10) défini par un axe de symétrie (AA') longitudinal, le support (10) comprenant une pluralité de moyens de diffusion d'au moins un stimulus sensoriel, lesdits moyens de diffusion comprenant des moyens de visualisation d'un stimulus visuel (101-136) et des moyens de génération d'un stimulus auditif adjacents aux moyens de visualisation d'un stimulus visuel, les moyens de diffusion étant arrangés de manière régulière selon des arcs d'iso-vergence (201-204) disposés le long du support (10) ;
- un système de commande (20) au moins destiné à commander la diffusion dudit au moins un stimulus sensoriel (101-136).

2. Dispositif selon la revendication précédente, comprenant plus de deux arcs d'iso-vergence, préférentiellement quatre arcs d'iso-vergence (201-204).

3. Dispositif selon l'une des revendications précédentes, dans lequel chaque arc d'iso-vergence (201-204) comprend au moins trois moyens de diffusion d'un stimulus sensoriel, préférentiellement neuf moyens de diffusion d'au moins un stimulus sensoriel.

4. Dispositif selon l'une des revendications précédentes dans lequel le support (10) est trapézoïdal ou triangulaire.

5. Dispositif selon l'une des revendications précédentes, dans lequel le support (10) est un éventail pliable.

6. Dispositif selon l'une des revendications 1 à 5, comprenant un premier arc d'iso-vergence (201) à une distance (d1) de 20 cm du patient, un second arc d'iso-vergence (202) à une distance (d2) de 40 cm du patient, un troisième arc d'iso-vergence (203) à une distance (d3) de 70 cm du patient, un quatrième arc d'iso-vergence (204) à une distance (d4) de 150 cm du patient.

7. Dispositif selon l'une des revendications 1 à 6, dans lequel les moyens de visualisation sont constitués de sources lumineuses émettant dans le domaine du visible, typiquement des diodes électroluminescentes.

8. Dispositif selon l'une des revendications précédentes, comprenant en outre des moyens d'acquisition (30) des mouvements des yeux du patient (P).

## Patentansprüche

1. Vorrichtung zur Rehabilitation und/oder zum Training der binokularen Motorik durch sensorische Stimulation eines Patienten, umfassend:
- einen ebenen Träger (10), der durch eine Symmetrielängsachse (AA') definiert ist, wobei der Träger (10) eine Vielzahl an Mitteln zur Aussendung wenigstens eines sensorischen Stimulus umfasst, wobei die Aussendungsmittel Mittel zur Visualisierung eines visuellen Stimulus (101-136) und Mittel zur Erzeugung eines auditiven Stimulus angrenzend an die Mittel zur Visualisierung eines visuellen Stimulus umfassen, wobei die Aussendungsmittel auf Isovergenz-Bögen (201-204) angeordnet sind, die entlang des Trägers (10) regelmäßig angeordnet sind;
- ein Steuerungssystem (20), welches zumindest dazu bestimmt ist, die Aussendung des wenigstens einen sensorischen Stimulus (101-136) zu steuern.

2. Vorrichtung gemäß dem vorherigen Anspruch, welche mehr als zwei Isovergenz-Bögen, bevorzugt vier Isovergenz-Bögen (201-204), umfasst.

3. Vorrichtung gemäß einem der vorherigen Ansprüche, wobei jeder Isovergenz-Bogen (201-204) wenigstens drei Mittel zur Aussendung eines sensorischen Stimulus, bevorzugt neun Mittel zur Aussendung wenigstens eines sensorischen Stimulus, umfasst.

4. Vorrichtung gemäß einem der vorherigen Ansprüche, wobei der Träger (10) trapezförmig oder dreieckig ist.

5. Vorrichtung gemäß einem der vorherigen Ansprüche, wobei der Träger (10) ein faltbarer Fächer ist.

6. Vorrichtung gemäß einem der Ansprüche 1 bis 5, welche einen ersten Isovergenz-Bogen (201) in einem Abstand (d1) von 20 cm vom Patienten, einen zweiten Isovergenz-Bogen (202) in einem Abstand (d2) von 40 cm vom Patienten, einen dritten Isovergenz-Bogen (203) in einem Abstand (d3) von 70 cm vom Patienten und einen vierten Isovergenz-Bogen (204) in einem Abstand (d4) von 150 cm vom Patienten umfasst.

7. Vorrichtung gemäß einem der Ansprüche 1 bis 6, wobei die Visualisierungsmittel von Lichtquellen, welche im sichtbaren Bereich aussenden, typischerweise Lumineszenzdioden, gebildet werden.

8. Vorrichtung gemäß einem der vorherigen Ansprüche, welche außerdem Mittel (30) zur Erfassung der Augenbewegungen des Patienten (P) umfasst.

## Claims

1. A device for rehabilitating and/or training binocular motivity by sensory stimulation of a patient including:
- a planar stand (10) defined by a longitudinal axis of symmetry (AA'), the stand (10) including a plurality of means of transmitting at least one sensory stimulus, said transmission means including means for displaying a visual stimulus (101-136) and means of transmitting an auditory stimulus adjacent to the means for displaying a visual stimulus, the transmission means being arranged regularly along isovergence arcs (201-204) positioned along the stand (10);
- a control system (20) designed to at least control the transmission of said at least one sensory stimulus (101-136).

2. The device according to the previous claim, including more than two isovergence arcs, preferably four isovergence arcs (201-209).

3. The device according to one of the previous claims, wherein each isovergence arc (201-204) includes at least three means of transmitting a sensory stimulus, preferably nine means of transmitting at least one sensory stimulus.

4. The device according to one of the previous claims, wherein the stand (10) is trapezoidal or triangular.

5. The device according to one of the previous claims, wherein the stand (10) is a foldable fan.

6. The device according to one of claims 1 to 5, including a first isovergence arc (201) at a distance (d1) of 20 cm from the patient, a second isovergence arc (202) at a distance (d2) of 40 cm from the patient, a third isovergence arc (203) at a distance (d3) of 70 cm from the patient, a fourth isovergence arc (204) at a distance (d4) of 150 cm from the patient.

7. The device according to one of claims 1 to 6, wherein the display means consist of light sources emitting in the visible band, typically light-emitting diodes.

8. The device according to one of the previous claims, further including means (30) of acquiring the eye movements of the patient (P).
